# EUROPEAN PATENT SPECIFICATION

(11) **EP 3 653 131 B1**
(45) Date of publication and mention of the grant of the patent: **14.07.2021**
(21) Application number: 19206597.7
(22) Date of filing: 31.10.2019
(51) Int. Cl.: A61B 8/08

(54) **ULTRASOUND DIAGNOSIS APPARATUS FOR DETERMINING ABNORMALITY OF FETAL HEART, AND OPERATING METHOD THEREOF**
ULTRASCHALLDIAGNOSEVORRICHTUNG ZUR BESTIMMUNG EINER ANOMALIE EINES FÖTALEN HERZENS UND BETRIEBSVERFAHREN DAFÜR
APPAREIL DE DIAGNOSTIC PAR ULTRASONS PERMETTANT DE DÉTERMINER UNE ANOMALIE DU COEUR FOETAL ET SON PROCÉDÉ DE FONCTIONNEMENT

(30) Priority: 15.11.2018 KR 20180141136
(43) Date of publication of application: 20.05.2020
(73) Proprietor: Samsung Medison Co., Ltd., Hongcheon-gun, Gangwon-do, 25108 (KR)
(72) Inventor: Park, Sungwook, Gyeonggi-do (KR); Lee, Jinyong, Gyeonggi-do (KR)
(74) Representative: Jacobs, Bart

(56) References cited:
- EP-A1- 2 783 635
- US-A1- 2010 099 987
- COMSTOCK CH: "Normal fetal heart axis and position", OBSTETRICS AND GYNECOLOGY, vol. 70, no. 2, 1 August 1987 (1987-08-01) , pages 255-259, XP055462637, US ISSN: 0029-7844
- NATHALIE JEANNE BRAVO-VALENZUELA ET AL: "Prenatal diagnosis of congenital heart disease: A review of current knowledge", INDIAN HEART JOURNAL, vol. 70, no. 1, 1 January 2018 (2018-01-01), pages 150-164, XP055682506, IN ISSN: 0019-4832, DOI: 10.1016/j.ihj.2017.12.005

## Description

The disclosure relates to an ultrasound diagnosis apparatus for determining abnormality of a fetal heart and an operating method thereof, and more particularly, to an ultrasound diagnosis apparatus for detecting the position of a heart in an ultrasound image of a fetus and determining abnormality of a fetal heart based on the position of the detected heart.

In an ultrasound system, an ultrasound signal generated by a transducer of an ultrasound probe is irradiated to a certain region of an object, an echo signal reflected from the region of the object is received to obtain an image of the region of the object. In particular, an ultrasound system is used for medical purposes such as observation of the inside of an object, detection of foreign materials, measurement of injury, or imaging of features.

Document US 2010/0099987 A1 discloses a method and an apparatus for determining abnormality of a heart of a fetus. For the image acquisition, the transducer has to positioned and moved according to a particular scheme. This leads to images where the heart is located in the vicinity of the upper left part, and the spine is located in the vicinity of the central lower part, i.e. where the left and right directions of the chest are already well-defined. ROIs including the spine and the heart are set. Based on the ROIs, volume scans comprising a plurality of images are performed. The spine can be automatically designated on the images, and based on the spine direction, e.g. a point P (cardiac position) and the inclination of the septum of the heart can be computed. A predetermined score for evaluating the abnormality of the heart is created.

This may be considered to form the closest prior art for the invention.

In identifying a health state of a fetus by analyzing an ultrasound image of the fetus, it is important to determine abnormality based on the position of a heart. When a heart is located on the left side in the chest in an ultrasound image of a fetal heart, the heart is classified as being normal. However, because the interpretation of an ultrasound image is currently dependent on a user only, for example, a medical doctor, determining abnormality of a heart of a fetus may be not only inconvenient, but also inaccurate, and thus abnormality of the heart may not be found.

Provided are an ultrasound diagnosis apparatus for detecting the position of a heart in an ultrasound image of a fetus and determining abnormality of a fetal heart based on the position of the detected heart, and an operating method thereof.

Provided are an ultrasound diagnosis apparatus for displaying information about abnormality of a fetal heart, and an operating method thereof.

Additional aspects will be set forth in part in the description which follows and, in part, will be apparent from the description, or may be learned by practice of the presented embodiments.

In accordance with an aspect of the disclosure, a method of determining abnormality of a heart of a fetus includes detecting a chest and a spine of the fetus in an ultrasound image of a fetal heart; receiving a user's input for inputting information regarding left and right directions of the chest of the fetus in the ultrasound image; detecting a position of the heart in the ultrasound image; and determining abnormality of the heart of the fetus based on the information about the left and right directions input by the user's input and information about the detected position of the heart.

In one embodiment, the determining of the abnormality of the heart of the fetus may include determining abnormality in a size of the heart of the fetus based on information about areas of the heart and the chest of the fetus in the ultrasound image.

In one embodiment, the determining of the abnormality in the size of the heart of the fetus may include calculating a ratio of an area of the heart to an area of the chest of the fetus in the ultrasound image; and determining that the size of the heart is abnormal when the calculated area ratio exceeds 1/3.

In one embodiment, the detecting of the position of the heart may include detecting a cardiac apex based on the detected heart position, and the determining of the abnormality of the heart of the fetus may include determining abnormality of the heart of the fetus based on information about the detected heart position and a position and a direction of the detected cardiac apex.

In one embodiment, the detecting of the position of the heart may include detecting the position of the heart in the ultrasound image through training of inputting the ultrasound image to a deep neural network (DNN)-based machine learning model.

In one embodiment, the above method may further include displaying a user interface (UI) indicating information about the abnormality of the heart of the fetus by using at least one of numbers, characters, graphs, images, and color.

In one embodiment, the displaying of the UI may include displaying a first UI that guides the position of the heart through a virtual line surrounding an outline of the detected heart.

In one embodiment, the displaying of the UI may include displaying a second UI that guides information about a ratio of the area of the detected heart and the area of the detected chest in a form of a graph.

In one embodiment, the displaying of the UI may include displaying a color map indicating, in color, a range of the size of the area of the heart corresponding to a normal range compared to the area of the detected chest, to overlap on the ultrasound image.

In one embodiment, the displaying of the UI may include displaying a graph of the heart size per week that indicates, on a graph, a ratio of the size of the heart of the fetus to the size of the chest according to pregnancy weeks, and the graph of the heart size per week may include a guideline regarding a value corresponding to a heart size in a normal range.

In one embodiment, the method may further include outputting a warning message when the heart of the fetus is determined to be abnormal.

In accordance with another aspect of the disclosure, an ultrasound diagnosis apparatus for determining abnormality of a heart of a fetus includes a user input interface configured to receive a user's input for inputting information regarding left and right directions of a chest of the fetus in an ultrasound image related to a fetal heart; and a controller configured to detect the chest and a spine of the fetus in the ultrasound image, detect a position of the heart in the ultrasound image, and determine abnormality of the heart of the fetus based on the information about the left and right directions input by the user's input and information about the detected position of the heart.

In one embodiment, the controller is further configured to determine abnormality in a size of the heart of the fetus based on information about areas of the heart and the chest of the fetus in the ultrasound image.

In one embodiment, the controller is further configured to calculate a ratio of an area of the heart to an area of the chest of the fetus in the ultrasound image and determine that the size of the heart is abnormal when the calculated area ratio exceeds 1/3.

In one embodiment, the controller is further configured to detect a cardiac apex based on the detected heart position and determine abnormality of the heart of the fetus based on information about the detected heart position and a position and a direction of the detected cardiac apex.

In one embodiment, the controller may include a machine learning module for detecting the position of the heart in the ultrasound image through training of inputting the ultrasound image to a deep neural network (DNN)-based machine learning model.

In one embodiment, the ultrasound diagnosis apparatus may further include a display displaying a user interface (UI) indicating information about the abnormality of the heart of the fetus by using at least one of numbers, characters, graphs, images, and color.

In one embodiment, the display may display a first UI that guides the position of the heart through a virtual line surrounding an outline of the detected heart.

In one embodiment, the display may display a second UI that guides information about a ratio of the area of the detected chest and the area of the detected heart in a form of a graph.

In one embodiment, the display may display a color map indicating, in color, a range of the size of the area of the heart corresponding to a normal range compared to the area of the detected chest, the color map being overlapped on the ultrasound image.

In one embodiment, the display may display a graph of the heart size per week that indicates, on a graph, a ratio of the size of the heart of the fetus to the size of the chest according to pregnancy weeks, and the graph of the heart size per week may include a guideline regarding a value corresponding to a heart size in a normal range.

In one embodiment, the display may display a warning message when the heart of the fetus is determined to be abnormal.

In accordance with another aspect of the disclosure, a computer program product includes a computer-readable storage medium, wherein the storage medium includes instructions, which, when executed by one or more processors, cause an apparatus to detect a chest and a spine of the fetus in an ultrasound image related to a fetal heart; receive, through a user input interface of the apparatus, a user's input for inputting information about left and right directions of the chest of the fetus in the ultrasound image; detect a position of the heart in the ultrasound image based on the information about the left and right directions input by the user's input; and determine abnormality of the heart of the fetus based on information about the detected position of the heart.

The above and other aspects, features, and advantages of certain embodiments of the disclosure will be more apparent from the following description taken in conjunction with the accompanying drawings, in which:
FIG. 1 is a conceptual diagram of an embodiment for obtaining an ultrasound image of a fetal heart, determining abnormality of the heart, and displaying information about the abnormality of the heart, by using an ultrasound diagnosis apparatus;
FIG. 2 illustrates a normal heart position and an abnormal heart position in an ultrasound image of a fetal heart;
FIG. 3 is a block diagram of constituent elements of an ultrasound diagnosis apparatus according to an embodiment of the disclosure;
FIG. 4 is a flowchart of a method of determining abnormality of a fetal heart, according to an embodiment of the disclosure;
FIG. 5 illustrates a method by which the ultrasound diagnosis apparatus according to an embodiment of the disclosure determines abnormality of a heart of a fetus based on the size of the heart;
FIG. 6 illustrates a method by which by the ultrasound diagnosis apparatus according to an embodiment of the disclosure detects the heart position and cardiac apex of a fetus in an ultrasound image of a fetal heart;
FIG. 7 illustrates a method by which the ultrasound diagnosis apparatus according to an embodiment of the disclosure detects a heart of a fetus in the ultrasound image of the heart by using machine learning;
FIGS. 8A to 8G illustrate examples of a user interface (UI) through which the ultrasound diagnosis apparatus according to an embodiment of the disclosure displays information about abnormality of a heart of a fetus;
FIGS. 9A and 9B illustrate examples of UIs through which the ultrasound diagnosis apparatus according to an embodiment of the disclosure displays information about abnormality of a heart in an ultrasound image of a fetal heart ;
FIGS. 10A and 10B illustrate examples of UIs through which the ultrasound diagnosis apparatus according to an embodiment of the disclosure displays information about abnormality of a heart of a fetus in an ultrasound image of the heart;
FIGS. 11A and 11B illustrate embodiments of displayed graphs indicating a ratio of the size of a heart to the size of a chest of a fetus for each pregnancy week;
FIG. 12 is a block diagram of a configuration of the ultrasound diagnosis apparatus according to an embodiment of the disclosure; and
FIGS. 13A to 13C illustrate the ultrasound diagnosis apparatus according to an embodiment of the disclosure.

Advantages and features of one or more embodiments of the present inventive concept and methods of accomplishing the same may be understood more readily by reference to the following detailed description of the embodiments and the accompanying drawings. In this regard, the present embodiments may have different forms and should not be construed as being limited to the descriptions set forth herein. Rather, these embodiments are provided so that this disclosure will be thorough and complete and will fully convey the concept of the present embodiments to one of ordinary skill in the art, and the present inventive concept will only be defined by the appended claims.

Terms used herein will now be briefly described and then one or more embodiments of the present inventive concept will be described in detail.

All terms including descriptive or technical terms which are used herein should be construed as having meanings that are obvious to one of ordinary skill in the art. However, the terms may have different meanings according to the intention of one of ordinary skill in the art, precedent cases, or the appearance of new technologies. Also, some terms may be arbitrarily selected by the applicant, and in this case, the meaning of the selected terms will be described in detail in the detailed description of the inventive concept. Thus, the terms used herein have to be defined based on the meaning of the terms together with the description throughout the specification.

When a part "includes" or "comprises" an element, unless there is a particular description contrary thereto, the part can further include other elements, not excluding the other elements. Also, the term "unit" in the embodiments of the present inventive concept means a software component or hardware component such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with the smaller number of components and "units", or may be divided into additional components and "units".

In the present specification, an "object" may be a human, an animal, or a part of a human or animal. For example, the object may be an organ (e.g., the liver, the heart, the womb, the brain, a breast, or the abdomen), a blood vessel, or a combination thereof. Furthermore, the "object" may be a phantom. The phantom means a material having a density, an effective atomic number, and a volume that are approximately the same as those of an organism. For example, the phantom may be a spherical phantom having properties similar to the human body.

Furthermore, in the present specification, a "user" may be, but is not limited to, a medical expert, such as a medical doctor, a nurse, a medical laboratory technologist, and a technician who repairs a medical apparatus.

Furthermore, in the present specification, an expression such as "first", "second" or "first-1" is an exemplary term to refer to a different constituent element, object, image, pixel, or patch. Accordingly, the expression "first", "second" or "first-1" does not represent an order between constituent elements or any priority.

Reference will now be made in detail to embodiments, examples of which are illustrated in the accompanying drawings. In this regard, the present embodiments may have different forms and should not be construed as being limited to the descriptions set forth herein. In the following description, well-known functions or constructions are not described in detail so as not to obscure the embodiments with unnecessary detail.

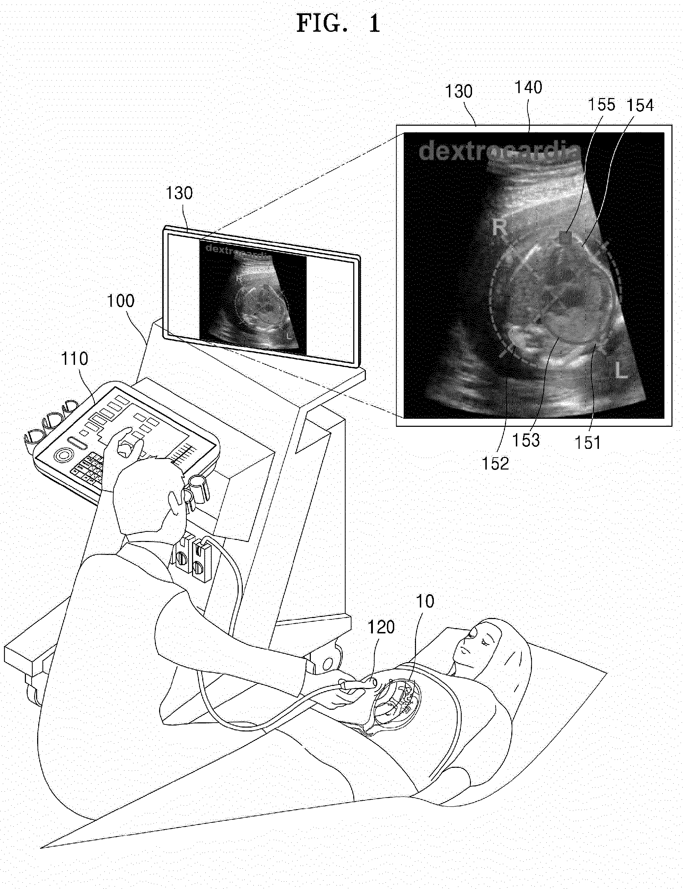

FIG. 1 is a conceptual diagram of a method of obtaining an ultrasound image of a fetal heart and determining abnormality of the fetal heart, by using an ultrasound diagnosis apparatus 100 according to an embodiment of the disclosure.

Referring to FIG. 1, a user 1 may obtain an ultrasound image 140 of a heart of a fetus 10 through a probe 120 of the ultrasound diagnosis apparatus 100. In an embodiment, the probe 120 may irradiate an ultrasound to the abdomen of a pregnant woman pregnant with the fetus 10, receive an ultrasound echo signal reflected from the abdomen of a pregnant woman, and perform signal processing on the received ultrasound echo signal to obtain the ultrasound image 140. The ultrasound diagnosis apparatus 100 may display, on a display 130, the ultrasound image 140.

In an embodiment, the ultrasound diagnosis apparatus 100 may detect the chest and spine of a fetus and the position of a heart from the ultrasound image 140, based on the position of the chest and spine. In an embodiment, the ultrasound diagnosis apparatus 100 may receive a user's input for inputting information regarding the left and right directions in the ultrasound image 140 through a user input interface 110. The ultrasound diagnosis apparatus 100 may detect the heart position of a fetus from the ultrasound image 140, and determine abnormality of a fetal heart based on the left and right direction information input by the user's input and the detected heart position. In detail, the ultrasound diagnosis apparatus 100 may identify the position of a heart with respect to the left and right directions of the chest set by the user's input received through the user input interface 110, and determine whether the identified heart position is normal or abnormal.

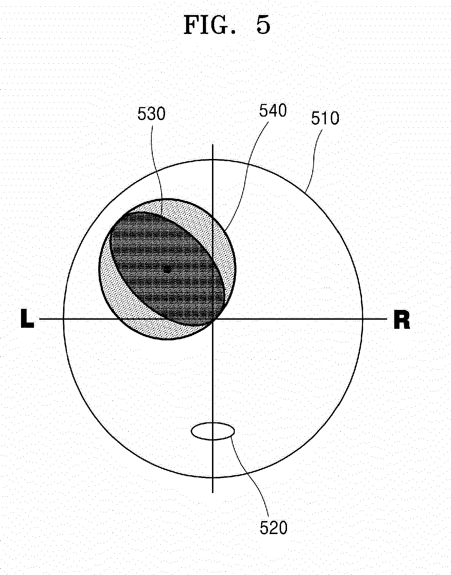

In an embodiment, the ultrasound diagnosis apparatus 100 may determine abnormality of a heart of a fetus based on not only the position of the heart, but also the size of the heart, which is described in detail with reference to FIG. 5.

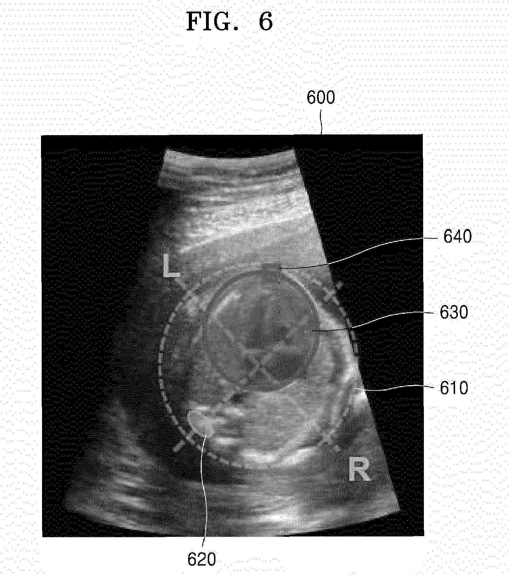

In an embodiment, the ultrasound diagnosis apparatus 100 may determine abnormality of a fetal heart based on the information about the position of a heart and the information about the position and direction of a cardiac apex, which is described in detail with reference to FIG. 6.

The ultrasound diagnosis apparatus 100 may display, on the display 130, user interfaces (UIs) 151 to 155 indicating information about the abnormality of a fetal heart by using at least one of numbers, characters, graphs, images, and colors. In an embodiment, the display 130 may display the ultrasound image 140 and the UIs 151 to 155 altogether. For example, the display 130 may display the UIs 151 to 155 to overlap on the ultrasound image 140.

The first UI 151 among the UIs 151 to 155 may indicate information regarding the left and right directions of the chest of a fetus. The left and right directions of the chest of a fetus in the ultrasound image 140 may be set by a user's input received through, but the present disclosure is not limited thereto. In an embodiment, the ultrasound diagnosis apparatus 100 may automatically identify information about the direction of the chest of a fetus in the ultrasound image 140 and display the identified left and right direction information on the display 130.

The second UI 152 may indicate the position of a spine. In an embodiment, the ultrasound diagnosis apparatus 100 may detect the spine position of a fetus in the ultrasound image 140 and display the detected spine of a fetus through the second UI 152.

The third UI 153 may indicate the heart position of a fetus. In an embodiment, the ultrasound diagnosis apparatus 100 may detect the heart position of a fetus in the ultrasound image 140 and display, on the display 130, the third UI 153 that guides the position of a heart through a virtual line surrounding the outline of the detected heart.

The fourth UI 154 may indicate the heart position of a fetus corresponding to a normal range. In an embodiment, the ultrasound diagnosis apparatus 100 may display, on the display 130, the fourth UI 154 that guides the position and area of a heart corresponding to the normal range based on the position and area of the chest detected from the ultrasound image 140 on the display 130. In an embodiment, the fourth UI 154 may be displayed in a color different from the third UI 153, but the present disclosure is not limited thereto. In an embodiment, the display 130 may display the fourth UI 154 to overlap on the ultrasound image 140.

The fifth UI 155 may indicate the position of the cardiac apex of a fetus. In the present specification, the "cardiac apex" may signify an edge of the left-front side located on a surface of the left ventricle of a heart. In an embodiment, the ultrasound diagnosis apparatus 100 may detect, from the ultrasound image 140, the position of the cardiac apex according to a ratio between the major axis and the minor axis of a heart and display, on the display 130, the fifth UI 155 that indicates the detected position of the cardiac apex.

According to the related art, because the interpretation of the ultrasound image related to a fetal heart is depend upon only the determination by a user such as a medical doctor or a clinical pathologist, accuracy and rapidity of the determination of the abnormality of a heart of a fetus may not be guaranteed and may be inconvenient. In the embodiment of FIG. 1, the ultrasound diagnosis apparatus 100 may detect the position of a heart in the ultrasound image 140 and automatically determine abnormality of a heart based on the detected heart position, thereby improving accuracy in the determining of health abnormality related to a fetal heart. Furthermore, the ultrasound diagnosis apparatus 100 may display, on the display 130, the UIs 151 to 155 that indicate information about the abnormality of a heart of the fetus 10, and thus a user may intuitively identify the abnormality of a fetal heart, thereby user convenience may be improved.

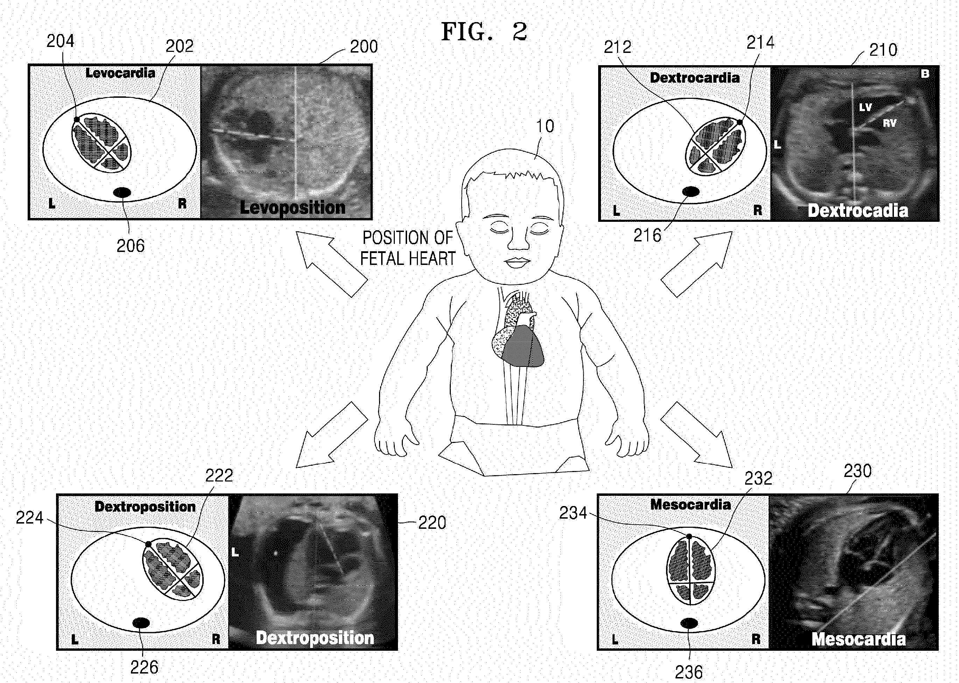

FIG. 2 illustrates a normal heart position and an abnormal heart position in an ultrasound image of a fetal heart.

Referring to FIG. 2, in an ultrasound image of the fetus 10, whether a heart is normal or abnormal may be determined based on the position of a heart in the chest. In an embodiment, the abnormality of a fetal heart may be determined based on not only the heart position of the fetus 10, but also the position and direction of the cardiac apex in an ultrasound image.

Among a plurality of ultrasound images 200 to 230 regarding the fetus 10, in the first ultrasound image 200, a heart 202 is located on the left side in the chest with respect to a spine 206, which means a case in which the position of a heart is normal. In the first ultrasound image 200, a cardiac apex 204 is located on the left side with respect to the spine 206 and tilts in the left direction. In this case, the heart of the fetus 10 may be determined to be at a normal position.

The second ultrasound image 210 to the fourth ultrasound image 230 may be ultrasound images corresponding to a case in which the heart position of the fetus 10 is abnormal. In the second ultrasound image 210, a heart 212 is located on the right side in the chest with respect to a spine 216, and a cardiac apex 214 is also located on the right side. The cardiac apex 214 tilts in the right direction in the chest with respect to the spine 216. As in the second ultrasound image 210, a case in which both of the heart 212 and the cardiac apex 214 are located on the right side and the cardiac apex 214 tilts in the right direction is referred to as dextrocardia.

In the third ultrasound image 220, a heart 222 is located on the right side in the chest with respect to a spine 226, but a cardiac apex 224 is located at the center portion of the chest similar to the position of the spine 226. In the third ultrasound image 220, the cardiac apex 224 tilts in the left direction. As in the third ultrasound image 220, a case in which the heart 222 is located on the right side, but the cardiac apex 224 tilts in the left direction is referred to as dextroposition.

In the fourth ultrasound image 230, a heart 232 and a cardiac apex 234 are located at the center portion in the chest with respect to a spine 236, and the cardiac apex 234 is located in the central direction. As in the fourth ultrasound image 230, a case in which the heart 232 is located at the center portion and the cardiac apex 234 is located in the central direction is referred to as mesocardia.

The information about the heart position and the position and direction of the cardiac apex in the ultrasound images 200 to 230 of FIG. 2 may become important parameters in determining abnormality of the heart of the fetus 10.

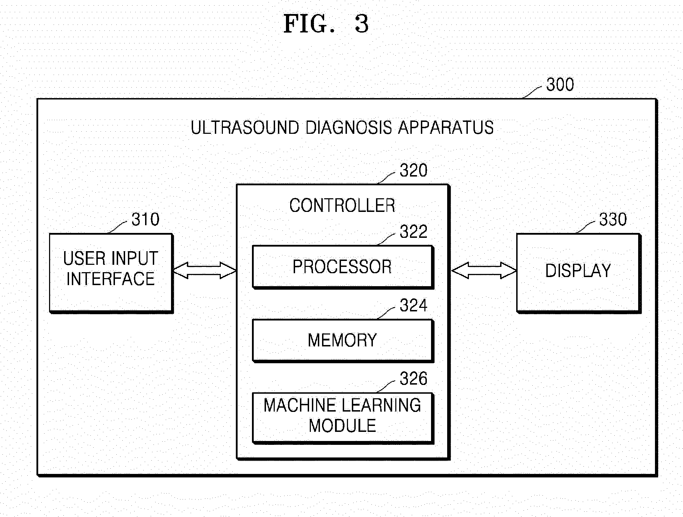

FIG. 3 is a block diagram of constituent elements of an ultrasound diagnosis apparatus 300 according to an embodiment of the disclosure. The ultrasound diagnosis apparatus 300 may be implemented in a cart type or a portable type. A portable ultrasound diagnosis apparatus may include a picture archiving and communication system (PACS) viewer, hand-carried cardiac ultrasound (HCU) equipment, a smart phone, a laptop computer, a personal digital assistance (PDA), a tablet PC, etc., but the present disclosure is not limited thereto.

In an embodiment, the ultrasound diagnosis apparatus 300 may be an apparatus for generating an ultrasound image by processing ultrasound image data received from an ultrasound probe, and displaying a generated image, or an apparatus for implementing only an image display function without a separate image processing function.

Referring to FIG. 3, the ultrasound diagnosis apparatus 300 may include a user input interface 310, a controller 320, and a display 330. However, the constituent elements illustrated in FIG. 3 are only essential constituent elements of the ultrasound diagnosis apparatus 300, and the ultrasound diagnosis apparatus 300 according to the present disclosure may include further constituent elements in addition to the illustrated constituent elements. In an embodiment, the ultrasound diagnosis apparatus 300 may further include at least one of a probe, a communicator, a storage, and an image processing unit.

The user input interface 310 may receive a user's input to control the ultrasound diagnosis apparatus 300. In an embodiment, the user input interface 310 may receive a use's input for inputting information regarding the left and right directions of the chest of a fetus in an ultrasound image related to a fetal heart. The user input interface 310 may include a hardware configuration, for example, a button, a key pad, a mouse, a trackball, a touch pad, a touch screen, a jog switch, etc., but the present disclosure is not limited thereto. When the display 330 is a touch screen, the user input interface 310 may be incorporated with the touch screen to receive a user's touch input.

The controller 320 may control the operations of the respective constituent elements of the ultrasound diagnosis apparatus 300. The controller 320 may include a processor 322, a memory 324, and a machine learning module 326. In an embodiment, the controller 320 may include the memory 324 for storing a program code or data to perform a certain function and the processor 322 for processing the program code and data stored in the memory 324. In an embodiment, the controller 320 may further include the machine learning module 326.

The controller 320 may be implemented in various combinations of one or more memories 324 and one or more processors 322. The processor 322 may generate and delete a program module according to an operation state of the ultrasound diagnosis apparatus 300 and process the operations of the program module. In an embodiment, the controller 320 may detect the chest and spine of a fetus in an ultrasound image, detect the position of a heart in the ultrasound image, and determine whether the position of a heart detected based on the left and right direction information input by the user's input received through the user input interface 310 is an abnormal position.

The processor 322 forming the controller 320 may detect a fetal heart in the ultrasound image through general purpose image processing technology or machine learning. For example, the processor 322 may include a hardware module including at least one of a central processing unit (CPU), a microprocessor, and a graphical processing unit (GPU). When the ultrasound diagnosis apparatus 300 is portable, for example, a smart phone, a laptop computer, PDA, or a table PC, the processor 322 may be implemented by an application processor (AP).

The memory 324 may be implemented by a hardware apparatus, for example, a random access memory (RAM) or a read only memory (ROM), for storing a program code or data to perform the respective functions of the ultrasound diagnosis apparatus 300, but the present disclosure is not limited thereto.

The controller 320 may determine abnormality in the size of a fetal heart based on the information about the area of a fetal heart in an ultrasound image. In an embodiment, the controller 320 may calculate a ratio of the size of the heart area to the size of the area of a chest of a fetus, and may determine that the size of a heart is abnormal when the calculated area ratio is 1/3 or more.

The controller 320 may detect a cardiac apex based on the detected heart position in an ultrasound image, and may determine abnormality of a fetal heart based on the information about the position and direction of the detected cardiac apex and the heart position information.

The machine learning module 326 may include a hardware unit with computation capability of performing algorithms and relevant applications of all machine learning models including an artificial neural network model, a support vector machine (SVM), and deep learning. In an embodiment, the machine learning module 326 may detect a heart, a cardiac apex, a spine, or a chest in an ultrasound image by using a machine learning model. The machine learning module 326 may detect positions of a heart, a cardiac apex, a spine, or a chest in an ultrasound image by using, for example, a well-known deep neural network (DNN) model such as a convolution neural network (CNN) model or a recurrent neural network (RNN) model. Although FIG. 3 illustrates the processor 322 as a separate constituent element from the machine learning module 326, the present disclosure is not limited to such a structure. In an embodiment, the processor 322 and the machine learning module 326 may be configured in a single chip, not in separate modules.

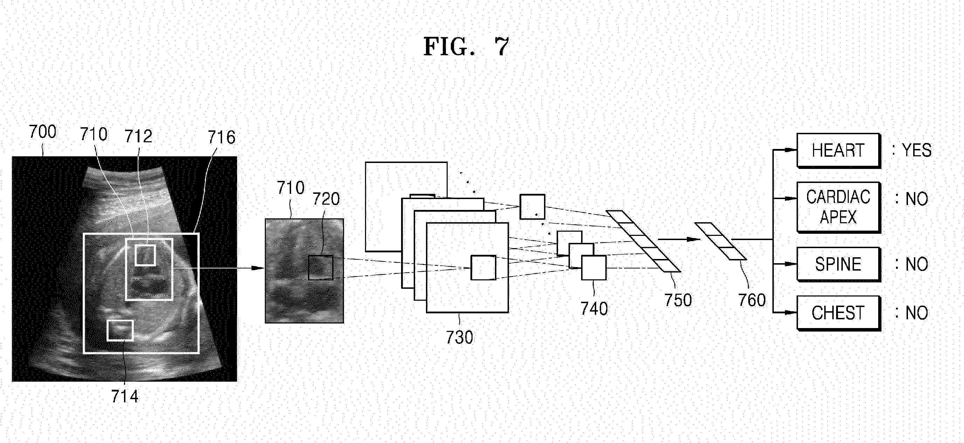

A method of detecting the position of a heart by using machine learning, by the machine learning module 326, is described in detail in FIG. 7.

The display 330 may display a UI indicating the information about the abnormality of a fetal heart obtained by the controller 320 by using at least one of numbers, characters, graphs, images, and color.

The display 330 may be configured with a physical apparatus including at least one of, for example, a LCD display, a PDP display, an OLED display, a FED display, a LED display, a VFD display, a digital light processing (DLP) display, a flat panel display, a 3D display, and a transparent display, but the present disclosure is not limited thereto. In an embodiment, the display 330 may be configured with a touch screen including a touch interface.

In an embodiment, the display 330 may display the UI to overlap on the ultrasound image of a fetus.

The display 330 may display a UI that guides the position of a heart using a virtual line surrounding the outline of a fetal heart detected by the controller 320 from the ultrasound image.

The display 330 may display a UI indicating information about a ratio of the size of a heart area to the size of a chest area of a fetus detected by the controller 320 from the ultrasound image, in the form of a graph. In an embodiment, the display 330 may display a color map indicating a range of the size of the heart area corresponding to the normal range with respect to the size of the detected chest area, to overlap on the ultrasound image of a fetal.

The display 330 may display a graph of the heart size per week indicating history information about the size of the heart area of a fetus according to pregnancy weeks. In an embodiment, the graph of the heart size per week may be a graph indicating a change of a heart size according to the pregnancy weeks by indicating a value of a ratio of the size of a heart area to the size of a chest area on the graph. In an embodiment, the display 330 may display a guideline regarding a value corresponding to the heart size in a normal range on the graph of the heart size per week.

The display 330 may output a warning message when the controller 320 determines that a fetal heart is abnormal. A warning message may be indicated by characters or images, but the present disclosure is not limited thereto.

In an embodiment, the ultrasound diagnosis apparatus 300 may include a speaker for outputting a warning message with voice or sound when a fetal heart is determined to be abnormal.

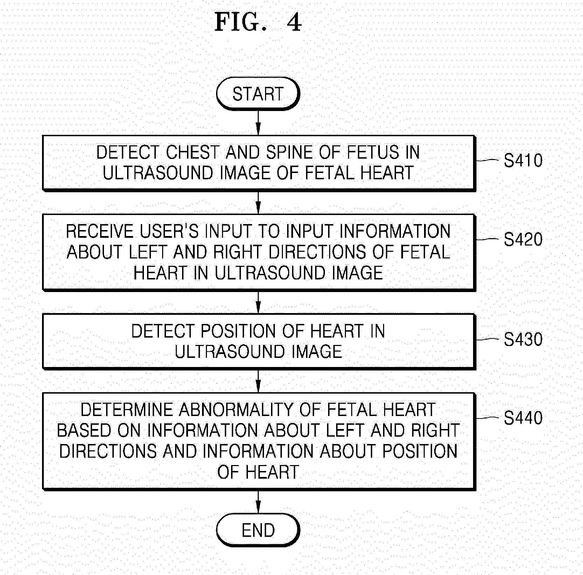

FIG. 4 is a flowchart of a method by which the ultrasound diagnosis apparatus determines abnormality of a fetal heart, according to an embodiment of the disclosure.

In S410, the ultrasound diagnosis apparatus detects the chest and spine of a fetus from the ultrasound image related to a fetal heart. In an embodiment, the ultrasound diagnosis apparatus may detect the chest and spine of a fetus in an ultrasound image by using general purpose image processing technology. In another embodiment, the ultrasound diagnosis apparatus may detect the positions of the spine and chest of a fetus in an ultrasound image by using the well-known DNN such as CNN or RNN.

In S420, the ultrasound diagnosis apparatus receives a user's input for inputting information regarding the left and right directions of the chest of a fetus in the ultrasound image. The ultrasound diagnosis apparatus may set the left and right directions of the heart in the chest in the ultrasound image based on the received user's input.

In some embodiments, S420 may be omitted. In other words, in an embodiment, the ultrasound diagnosis apparatus may automatically identify the left and right directions of the heart of a fetus in the ultrasound image of a fetal without a user's input, and set the left and right directions.

In S430, the ultrasound diagnosis apparatus detect the position of a heart in an ultrasound image. In an embodiment, the ultrasound diagnosis apparatus may detect the position of a heart through artificial intelligence learning using the general purpose image processing technology or machine learning. In an embodiment, the ultrasound diagnosis apparatus may determine where the heart is located with respect to the chest and spine based on the left and right directions of the chest determined by the user's input received in S420.

In an embodiment, the ultrasound diagnosis apparatus may detect the position of a cardiac apex according to a ratio of the major axis and the minor axis of a heart in the ultrasound image of a fetus, and obtain information about the detected position of the cardiac apex and the tilt direction of the cardiac apex.

In S440, the ultrasound diagnosis apparatus determines abnormality of a fetal heart based on the left and right direction information and the heart position. In an embodiment, the ultrasound diagnosis apparatus may determine that a fetal heart is normal when in S430 the detected heart position is located on the left side of the chest with respect to the spine. In this case, whether it is in the left direction or the right direction may be determined by the user's input received in S420. In an embodiment, the ultrasound diagnosis apparatus may determine abnormality of a fetal heart based on the information not only about the position of a heart, but also about the position of the cardiac apex and the tilt direction of the cardiac apex.

FIG. 5 illustrates a method by which the ultrasound diagnosis apparatus determines abnormality of a heart of a fetus based on the size of the heart, according to an embodiment of the disclosure.

Referring to FIG. 5, the ultrasound diagnosis apparatus may detect boundaries of a chest 510, a spine 520, and a heart 530 in an ultrasound image of a fetus. In an embodiment, the ultrasound diagnosis apparatus may detect the chest 510, the spine 520, and the heart 530 in the ultrasound image by using the well-known image processing technology. In an embodiment, the ultrasound diagnosis apparatus may detect the chest 510, the spine 520, and the heart 530 in the ultrasound image by performing algorithms and relevant applications of all machine learning models including artificial neural network model, SVM, and deep learning.

The ultrasound diagnosis apparatus may determine abnormality of a fetal heart based on a ratio of the size of the area of the detected heart 530 to the size of the area of the detected chest 510. In an embodiment, the ultrasound diagnosis apparatus may calculate a ratio of the size of the area of the heart 530 to the size of the area of the chest 510 of fetus detected from in the ultrasound image, and determine that the fetal heart size is abnormal when the calculated area ratio exceeds 1/3. In an embodiment, the ultrasound diagnosis apparatus may determine that the fetal heart size is abnormal when the calculated area ratio is less than 1/3.

The ultrasound diagnosis apparatus may display a UI 540 indicating the size of a heart corresponding to the normal range according to a ratio of the size of the area of the heart 530 to the size of the area of the chest 510 of fetus. The UI 540 may indicate a boundary of the size of a heart corresponding to the normal range with respect to the size of the detected heart 530. The user may intuitively identify the size of a heart corresponding to the normal range, through UI 540 of FIG. 5.

FIG. 6 illustrates a method by which the ultrasound diagnosis apparatus detects a heart 630 and a cardiac apex 640 of a fetus in an ultrasound image 600 of a fetal heart, according to an embodiment of the disclosure.

Referring to FIG. 6, the ultrasound diagnosis apparatus may detect boundaries of a chest 610, a spine 620, and the heart 630 in the ultrasound image 600. The ultrasound diagnosis apparatus may detect the chest 610, the spine 620, and the heart 630 in the ultrasound image 600 by using the well-known image processing technology and machine learning.

The ultrasound diagnosis apparatus may detect the position of the cardiac apex 640 based on the ratio of the major axis and the minor axis of the detected heart 630 in the ultrasound image 600. In an embodiment, the ultrasound diagnosis apparatus may detect the cardiac apex 640 in the ultrasound image 600 by performing algorithms and relevant applications of all machine learning models including artificial neural network model, SVM, and deep learning.

The ultrasound diagnosis apparatus may determine abnormality of the heart 630 of a fetus based on the information about the position and direction of the detected cardiac apex 640 and information about the position of the detected heart 630. In detail, the ultrasound diagnosis apparatus may determine that the heart 630 of a fetus is normal when the detected heart 630 and the detected cardiac apex 640 are located on the left side with respect to the spine 620 in the chest 610, and the cardiac apex 640 tilts in the left direction. Otherwise, that is, when the heart 630 and the cardiac apex 640 are located on the right side or at the center with respect to the spine 620, or the cardiac apex 640 tilts in the right direction or is located in the central direction, the ultrasound diagnosis apparatus may determine that the heart 630 of a fetus is abnormal.

FIG. 7 illustrates a method by which the ultrasound diagnosis apparatus detects a heart of a fetus in an ultrasound image 700 by using machine learning, according to an embodiment of the disclosure.

Referring to FIG. 7, the ultrasound diagnosis apparatus trains an image of a heart in the ultrasound image 700 of a fetus by using CNN and detects the heart by using the trained neural network model, but such a method is presented for convenience of explanation. In an embodiment, the ultrasound diagnosis apparatus may detect a heart, a cardiac apex, a spine, or a chest in the ultrasound image by using the well-known DNN such as CNN or RNN. In an embodiment, the ultrasound diagnosis apparatus may detect a heart in the ultrasound image 700 by using machine learning such as SVM.

To detect a heart in the ultrasound image 700, the ultrasound diagnosis apparatus first extract an image region having an anatomical structure in the ultrasound image 700 of a fetus. In an embodiment, the ultrasound diagnosis apparatus may extract a first image region 710 including a heart in the ultrasound image 700, a second image region 712 including a cardiac apex, a third image region 714 including a spine, and a fourth image region 716 including a chest. The image extraction method may use well-known image processing technology, but the present disclosure is not limited thereto.

The ultrasound diagnosis apparatus may generate a feature map including a plurality of layers 730 through a convolution operation in which a filter 720 having a certain size is applied to a pixel value of the first image region 710 including a heart. The feature map may include the layers 730 having a feature vector value generated through a convolution operation in which the filter 720 is multiplied to the pixel value of the first image region 710, and after the filter 720 strides, the filter 720 is multiplied to other pixel value.

The ultrasound diagnosis apparatus may obtain a plurality of feature values 740 through a subsampling or pooling of a feature vector value included in the layers 730. A max pooling method may be used as the pooling method used in the process, but the present disclosure is not limited thereto.

The ultrasound diagnosis apparatus may obtain an output value 760 through a fully connected layer 750 that connects the obtained feature values 740 in one layer. The ultrasound diagnosis apparatus may identify that the output value 760 signifies a heart by comparing the obtained output value 760 with a previously trained label value.

The CNN model used in FIG. 7 may be a model using a network including a filter value obtained through training of detecting a heart from several hundreds or thousands of ultrasound images. Although FIG. 7 illustrates only a method of detecting a heart from the ultrasound image 700 by using a CNN model, a cardiac apex, a spine, or a chest may be detected by the same method.

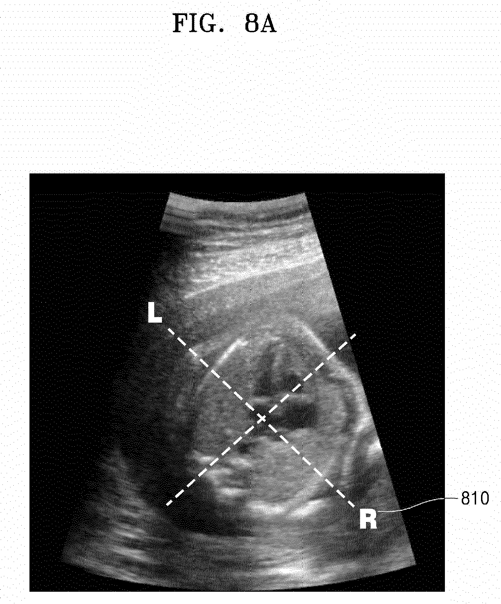

FIGS. 8A to 8G illustrate examples of a UI through which the ultrasound diagnosis apparatus according to an embodiment of the disclosure displays information about abnormality of a heart of a fetus. The ultrasound diagnosis apparatus may display a UI indicating information about the abnormality of a fetal heart in an ultrasound image of a fetal by using at least one of numbers, characters, graphs, images, and color.

Referring to FIG. 8A, the ultrasound diagnosis apparatus may display a first UI 810 indicating the information about the left and right directions of the chest of a fetus detected from the ultrasound image. The direction information indicated by the first UI 810 may be a UI indicating L (left) and R (right) by using a character, in which the left and right directions may be directly input by a user, but the present disclosure is not limited thereto. In an embodiment, the ultrasound diagnosis apparatus may automatically identify information about the direction of the chest of a fetus in an ultrasound image, and display the first UI 810 indicating the identified left and right direction information.

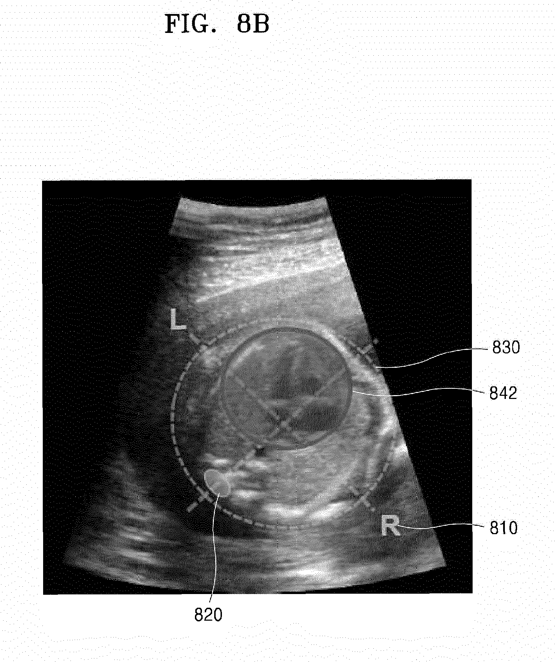

Referring to FIG. 8B, the ultrasound diagnosis apparatus may display a second UI 820 indicating the position of a spine and a third UI 830 indicating the boundary of a chest, in addition to the first UI 810 indicating the left and right directions of the chest of a fetus. In an embodiment, the ultrasound diagnosis apparatus may display a heart size guideline UI 842 that guides the range of a heart area corresponding to the normal range compared to the chest area. The heart size guideline UI 842 may be a UI that anticipates a region where a fetal heart is located based on the position of a chest and a spine and guides a range corresponding to 1/3 of the chest area in the anticipated region. Although FIG. 8B illustrates the heart size guideline UI 842 to be a blue circular shape, and the inside of the circle is filled with blue, this is exemplary and the present disclosure is not limited thereto.

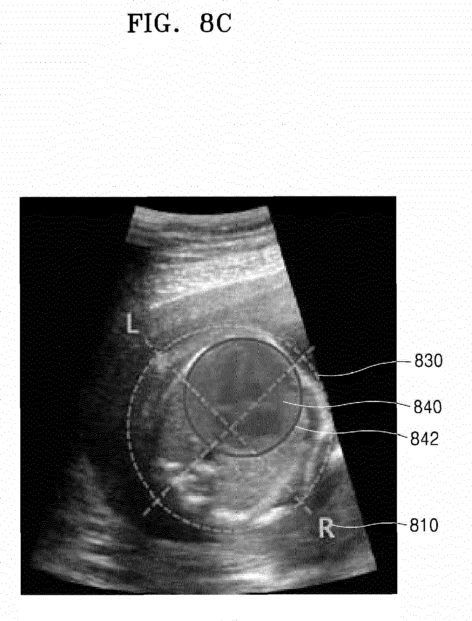

Referring to FIG. 8C, the ultrasound diagnosis apparatus may further display a fourth UI 840 indicating the position of a heart that is actually detected and the heart size guideline UI 842, in addition to the first UI 810, the second UI 820, and the third UI 830. The fourth UI 840 indicating the position of a heart may be displayed in a color different from the heart size guideline UI 842.

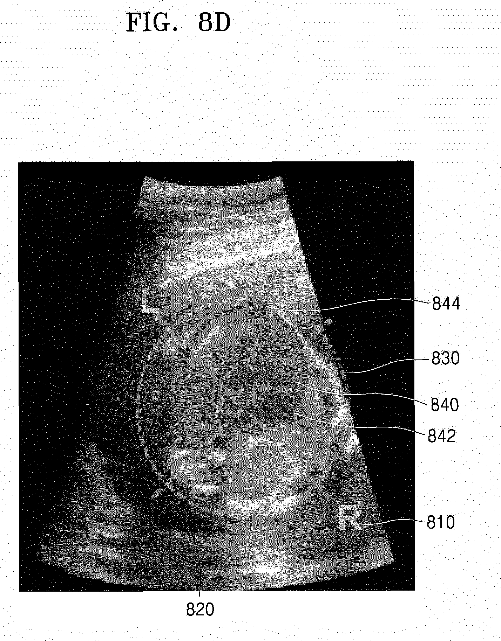

Referring to FIG. 8D, the ultrasound diagnosis apparatus may further display a cardiac apex UI 844 indicating the position of the cardiac apex, in addition to the first UI 810, the second UI 820, the third UI 830, the fourth UI 840, and the heart size guideline UI 842. The ultrasound diagnosis apparatus may detect the position of the cardiac apex based on the ratio of the major axis and the minor axis of a heart, and display the cardiac apex UI 844 indicating the detected position of the cardiac apex.

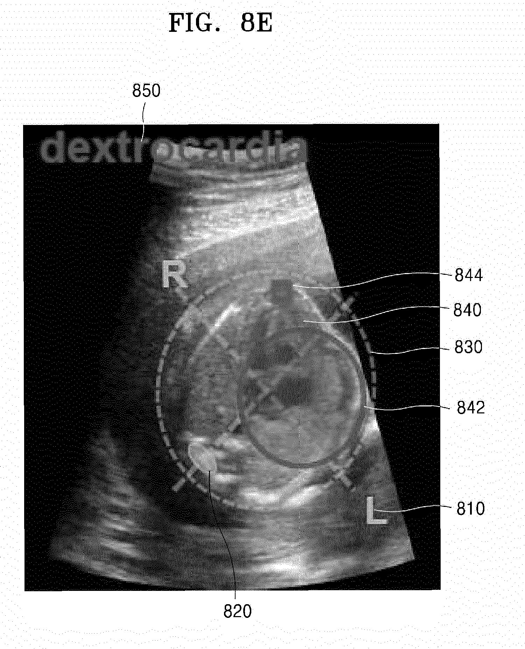

Referring to FIG. 8E, the ultrasound diagnosis apparatus may further display a fifth UI 850 indicating information about the abnormality of a fetal heart, in addition to the first UI 810, the second UI 820, the third UI 830, the fourth UI 840, the heart size guideline UI 842, and the cardiac apex UI 844. The fifth UI 850 may be a user interface indicating information about abnormality in the position or the size of the heart area of a fetus by using a character or an image. The fifth UI 850 may display information about not only normality or abnormality of a fetal heart, but also a type of abnormality when the fetal heart is abnormal. In the embodiment illustrated in FIG. 8E, the fetal heart and the cardiac apex are located on the right side of the chest, and the tilt direction of the cardiac apex is in the right direction, which corresponds to dextrocardia, and the fifth UI 850 may display characters of "dextrocardia" at one side of the ultrasound image.

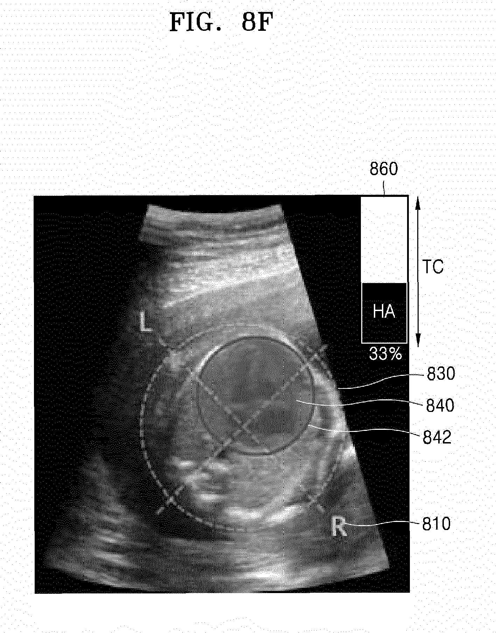

Referring to FIG. 8F, the ultrasound diagnosis apparatus may further display an area ratio UI 860 indicating information about a ratio of the size of the heart area compared to the size of the chest area, in addition to the first UI 810, the third UI 830, the fourth UI 840, and the heart size guideline UI 842. The area ratio UI 860 may be a UI indicating a value obtained by dividing the size of the heart area detected by the ultrasound diagnosis apparatus by the size of the chest area, in the form of a graph. In an example of FIG. 8F, the area ratio UI 860 may include characters TC indicating a chest, a bar indicating the size of a chest area, HA denoting the heart area, and a bar indicating the size of a heart area. In an embodiment, the area ratio UI 860 may display a calculated ratio, that is, a ratio value indicating the size of a heart area compared to the size of a chest area.

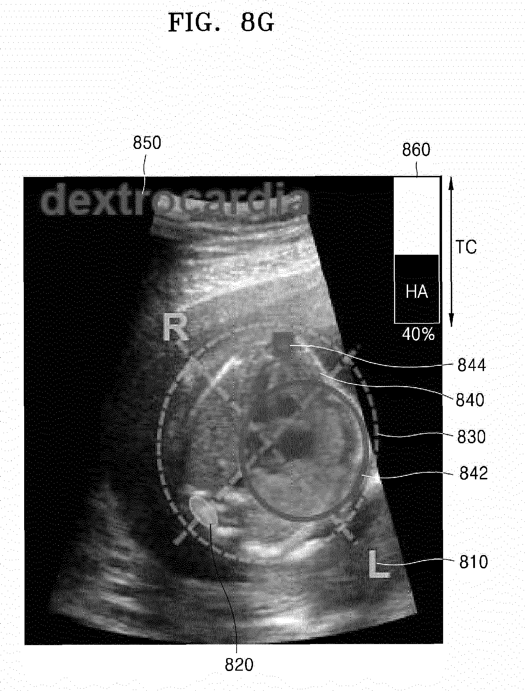

Referring to FIG. 8G, the ultrasound diagnosis apparatus may display the first UI 810, the second UI 820, the third UI 830, the fourth UI 840, the fifth UI 850, the heart size guideline UI 842, the cardiac apex UI 844, and the area ratio UI 860.

The UIs illustrated in FIGS. 8A to 8G are exemplary for convenience of explanation, and the UIs 810, 820, 830, 840, 842, 844, 850, and 860 illustrated in FIGS. 8A to 8G may be displayed in any combination that is not illustrated in the drawings. For example, the ultrasound diagnosis apparatus may display only the first UI 810 and the area ratio UI 860 in the ultrasound image.

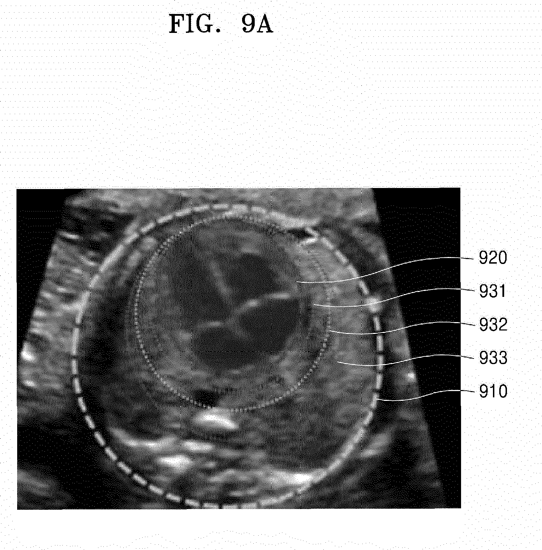

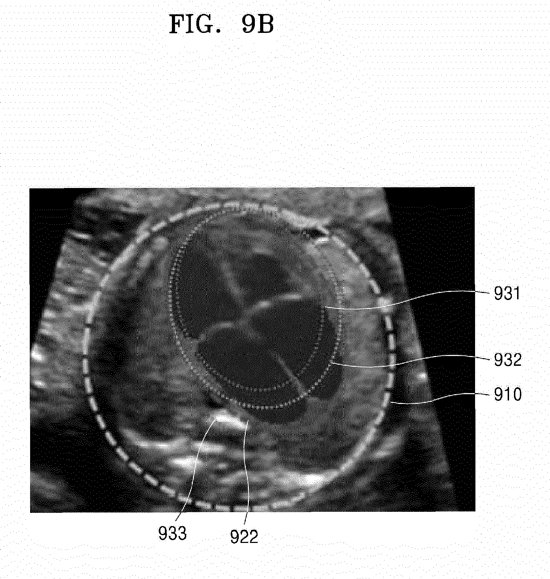

FIGS. 9A and 9B illustrate examples of UIs through which the ultrasound diagnosis apparatus according to an embodiment of the disclosure displays information about the abnormality of a heart in an ultrasound image of a fetal heart.

Referring to FIG. 9A, the ultrasound diagnosis apparatus may display guidelines regarding the size of a heart corresponding to the normal range compared to the size of a chest area of a fetus in the ultrasound image and the position of the heart. The display 330 (see FIG. 3) of the ultrasound diagnosis apparatus may display a first UI 910 indicating the position and area of the chest of a fetus and a second UI 920 indicating the position and area of a heart of the fetus, which are detected in the ultrasound image.

The ultrasound diagnosis apparatus may display a plurality of guidelines 931 to 933 regarding the size of a fetal heart corresponding to normality or abnormality compared to the chest area of a fetus detected in the ultrasound image. In an embodiment, the ultrasound diagnosis apparatus may calculate a ratio of the major axis and the minor axis of a heart of a fetus detected in an ultrasound image, and display, on a display, the guidelines 931 to 933 that guide the size of a heart corresponding to normality or abnormality based on the calculated ratio. In an embodiment, the guidelines 931 to 933 may be guidelines extending from a cardiac apex portion of a fetal heart.

For example, the first guideline 931 may be a guideline that guides a heart area of the normal range corresponding to 1/3 of the chest area, that is, the area indicated by the first UI 910. The second guideline 932 may be a guideline that guides a heart area that exceeds 1/3 of the area of the detected chest, but nor classified as an abnormal range. The third guideline 933 may be a guideline that guides a heart area that exceeds 1/3 of the area of the detected chest and corresponds to an abnormal range.

The first to third guidelines 931 to 933 may be displayed in different colors. For example, the first guideline 931 may be displayed in green meaning a normal heart size. The second guideline 932 may be displayed in yellow meaning a warning because the size of the heart is not classified to be abnormal but exceeds 1/3 of the chest area. The third guideline 933 may be displayed in red meaning that the size of a heart is classified to be abnormal. However, the colors of the first to third the guidelines 931 to 933 are exemplary, and the present disclosure is not limited to the illustration of FIG. 9A.

The second UI 920 indicating the position and area of a heart of a fetus detected in the ultrasound image may be displayed in a different color according to the size of the chest area. Referring to FIG. 9B altogether, the second UI 920, when having the size of a heart corresponding to the normal range compared to the chest area, may be displayed in blue, but when the size of a heart area exceeds 1/3 of the chest area, the second UI 922 may be displayed in red.

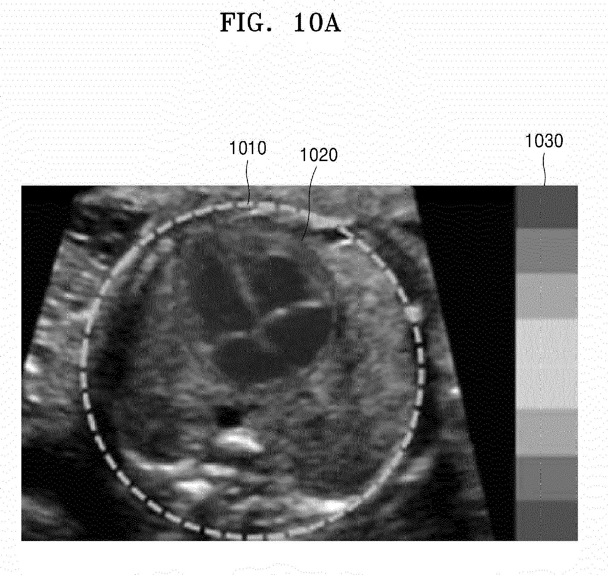

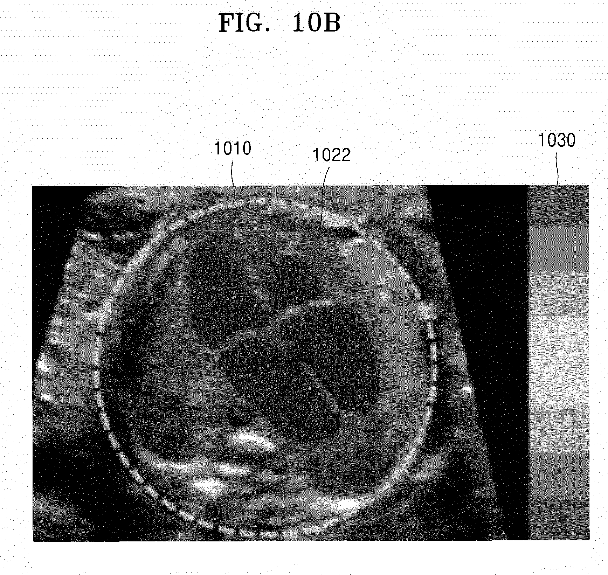

FIGS. 10A and 10B illustrate examples of UIs through which the ultrasound diagnosis apparatus according to an embodiment of the disclosure displays information about the abnormality of a heart of a fetus in the ultrasound image of the heart.

Referring to FIG. 10A, the ultrasound diagnosis apparatus may display a first UI 1010 indicating the position and area of a chest of a fetus and a second UI 1020 indicating the position and area of a heart of a fetus, which are detected in the ultrasound image. In an embodiment, the ultrasound diagnosis apparatus may display a color map 1030 indicating the range of the size of the heart area corresponding to the normal range compared to the area of the detected chest, in different colors, to overlap on the ultrasound image. In an embodiment, the color map 1030 may include a color scheme of colors from blue to red with different brightness and saturation, but the present disclosure is not limited thereto.

The second UI 1020 may be displayed being mapped with a color defined by the color map 1030 according to the size of a heart of a fetus detected in the ultrasound image. In an embodiment, when the second UI 1020 indicates the size of a heart corresponding to the normal range compared to the chest of a fetus, the second UI 1020 may be mapped in blue and displayed in blue.

Referring to FIG. 10B, when the size of a fetal heart detected in the ultrasound image corresponds to the abnormal range, a second UI 1022 may be mapped with red and displayed in a red color. In an embodiment, when the size of the heart area of a fetus exceeds 1/3 of the chest area, the heart may be classified to be abnormal, and the second UI 1022 may be displayed in red.

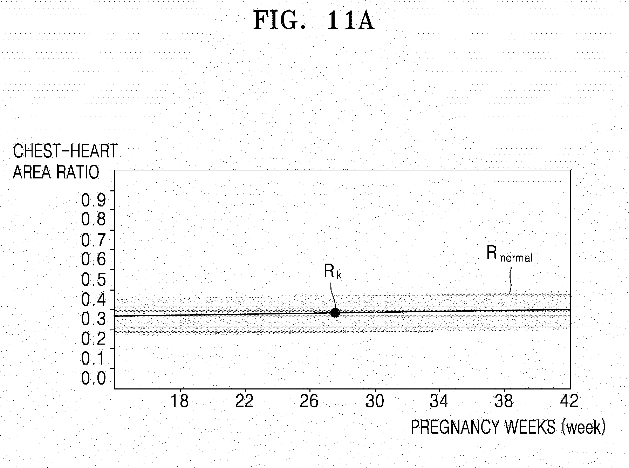

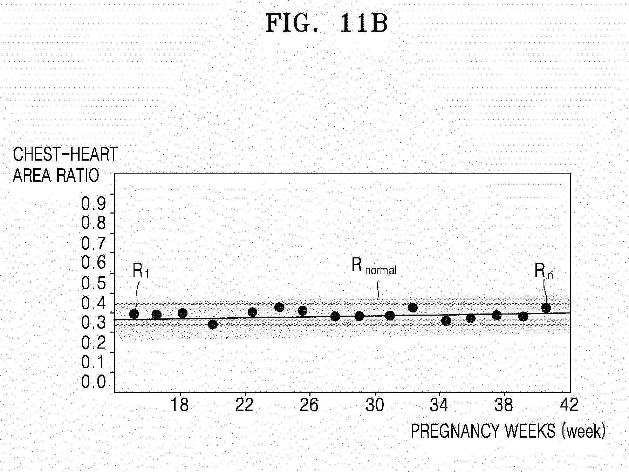

FIGS. 11A and 11B illustrate embodiments of displaying graphs indicating a ratio of the size of a heart to the size of a chest of a fetus for each pregnancy week.

Referring to FIG. 11A, the ultrasound diagnosis apparatus may display a graph of the heart size per week that indicates, on a graph, a ratio of the size of a fetal heart to the size of a fetal chest detected in an ultrasound image according to pregnancy weeks when the ultrasound image is captured. The graph of the heart size per week may be a graph indicating, on an x-axis, a pregnancy week when a pregnant woman has capturing of an ultrasound image and, on a y-axis, a value of a ratio obtained by dividing the size of the area of a heart of a fetus detected in the captured ultrasound image by the size of the area of a chest of a fetus. Referring to the graph illustrated in FIG. 11A, a ratio value Rₖ may signify a value when the size of the heart area of a fetus is about 0.34 times of the size of the chest area of a fetus detected in an ultrasound image captured at the 27^{th} pregnancy week.

In an embodiment, the graph of the heart size per week may further display a guideline Rₙₒᵣₘₐₗ regarding a ratio value corresponding to the size of a heart corresponding to the normal range. The guideline Rₙₒᵣₘₐₗ may indicate a range of a ratio value of the size of the heart area of a fetus corresponding to the normal range to the size of the chest area of a fetus detected in the ultrasound image. The guideline Rₙₒᵣₘₐₗ may be a value varying according to the pregnancy week, and displayed to roughly have a range of greater than or equal to 0.3 and less than 0.4.

Referring to FIG. 11B, the ultrasound diagnosis apparatus may display a graph of the heart size per week indicating history information of the size of a fetal heart detected according to the pregnancy week when a pregnant woman has capturing of an ultrasound image. Referring to the graph of the heart size per week, a pregnancy week when a pregnant woman has capturing of an ultrasound image may be indicated on an x-axis, and a value of a ratio obtained by dividing the size of the area of a heart of a fetus detected in the captured ultrasound image by the size of the area of a chest of a fetus may be indicated on a y-axis. Referring to the graph illustrated in FIG. 11B, ultrasound images are captured at the 16^{th}, 17^{th}, 19^{th}, ..., 41^{st} weeks, and the history information of ratio values R₁ to Rₙ regarding the size of a heart to the size of a chest of a fetus detected in the captured ultrasound image may be displayed.

Like the case of FIG. 11A, the graph of the heart size per week of FIG. 11B may also display the guideline Rₙₒᵣₘₐₗ regarding the ratio value corresponding to the size of a heart corresponding to the normal range.

Through the heart size graphs per week illustrated in FIGS. 11A and 11B, not only a user, but also a pregnant woman may intuitively identify whether the size of a fetal heart is normal, and thus user convenience may be improved.

As described above, according to an embodiment of the present disclosure, abnormality of a fetal heart may be automatically determined by analyzing an ultrasound image of a fetal heart, and accuracy and user convenience in diagnosis may be improved by displaying information about the abnormality of a heart.

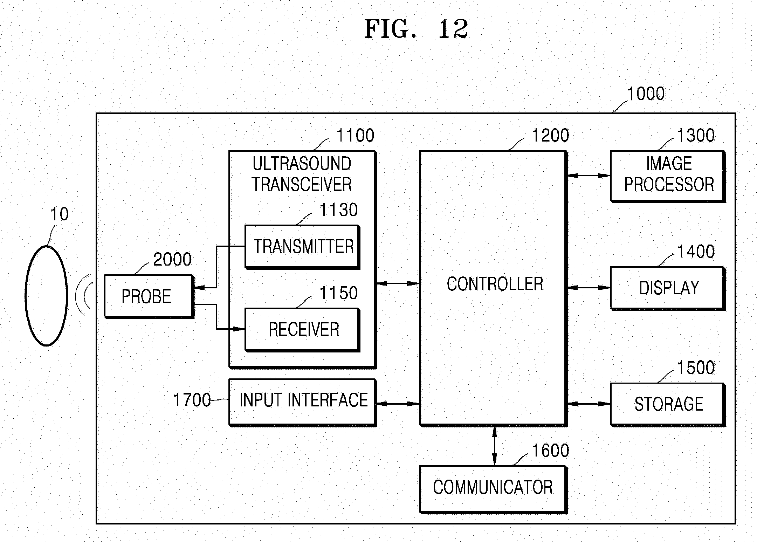

FIG. 12 is a block diagram illustrating a configuration of an ultrasound diagnosis apparatus 1000, i.e., a diagnostic apparatus, according to an exemplary embodiment.

Referring to FIG. 12, the ultrasound diagnosis apparatus 1000 may include a probe 2000, an ultrasound transceiver 1100, a controller 1200, an image processor 1300, one or more displays 1400, a storage 1500, e.g., a memory, a communicator 1600, i.e., a communication device or an interface, and an input interface 1700.

The ultrasound diagnosis apparatus 1000 may be a cart-type or portable-type ultrasound diagnosis apparatus that is portable, moveable, mobile, or hand-held. Examples of the portable-type ultrasound diagnosis apparatus 1000 may include a smart phone, a laptop computer, a personal digital assistant (PDA), and a tablet personal computer (PC), each of which may include a probe and a software application, but embodiments are not limited thereto.

The probe 2000 may include a plurality of transducers. The plurality of transducers may transmit ultrasound signals to an object 10 in response to transmitting signals received by the probe 2000, from a transmitter 1130. The plurality of transducers may receive ultrasound signals reflected from the object 10 to generate reception signals. In addition, the probe 2000 and the ultrasound diagnosis apparatus 1000 may be formed in one body (e.g., disposed in a single housing), or the probe 2000 and the ultrasound diagnosis apparatus 1000 may be formed separately (e.g., disposed separately in separate housings) but linked wirelessly or via wires. In addition, the ultrasound diagnosis apparatus 1000 may include one or more probes 2000 according to embodiments.

The controller 1200 may control the transmitter 1130 for the transmitter 1130 to generate transmitting signals to be applied to each of the plurality of transducers based on a position and a focal point of the plurality of transducers included in the probe 2000.

The controller 1200 may control an ultrasound receiver 1150 to generate ultrasound data by converting reception signals received from the probe 2000 from analogue to digital signals and summing the reception signals converted into digital form, based on a position and a focal point of the plurality of transducers.

The image processor 1300 may generate an ultrasound image by using ultrasound data generated from the ultrasound receiver 1150.

The display 1400 may display a generated ultrasound image and various pieces of information processed by the ultrasound diagnosis apparatus 1000. The ultrasound diagnosis apparatus 1000 may include two or more displays 1400 according to the present exemplary embodiment. The display 1400 may include a touch screen in combination with a touch panel.

The controller 1200 may control the operations of the ultrasound diagnosis apparatus 1000 and flow of signals between the internal elements of the ultrasound diagnosis apparatus 1000. The controller 1200 may include a memory for storing a program or data to perform functions of the ultrasound diagnosis apparatus 1000 and a processor and/or a microprocessor (not shown) for processing the program or data. For example, the controller 1200 may control the operation of the ultrasound diagnosis apparatus 1000 by receiving a control signal from the input interface 1700 or an external apparatus.

The ultrasound diagnosis apparatus 1000 may include the communicator 1600 and may be connected to external apparatuses, for example, servers, medical apparatuses, and portable devices such as smart phones, tablet personal computers (PCs), wearable devices, etc., via the communicator 1600.

The communicator 1600 may include at least one element capable of communicating with the external apparatuses. For example, the communicator 1600 may include at least one among a short-range communication module, a wired communication module, and a wireless communication module.

The communicator 1600 may receive a control signal and data from an external apparatus and transmit the received control signal to the controller 1200 so that the controller 1200 may control the ultrasound diagnosis apparatus 1000 in response to the received control signal.

The controller 1200 may transmit a control signal to the external apparatus via the communicator 1600 so that the external apparatus may be controlled in response to the control signal of the controller 1200.

For example, the external apparatus connected to the ultrasound diagnosis apparatus 1000 may process the data of the external apparatus in response to the control signal of the controller 1200 received via the communicator 1600.

A program for controlling the ultrasound diagnosis apparatus 1000 may be installed in the external apparatus. The program may include command languages to perform part of operation of the controller 1200 or the entire operation of the controller 1200.

The program may be pre-installed in the external apparatus or may be installed by a user of the external apparatus by downloading the program from a server that provides applications. The server that provides applications may include a recording medium where the program is stored.

The storage 1500 may store various data or programs for driving and controlling the ultrasound diagnosis apparatus 1000, input and/or output ultrasound data, ultrasound images, applications, etc.

The input interface 1700 may receive a user's input to control the ultrasound diagnosis apparatus 1000 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

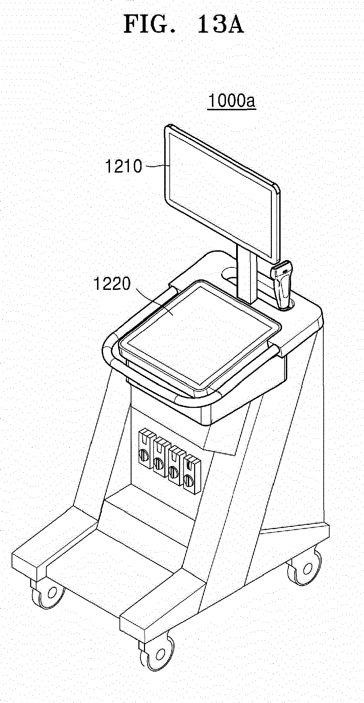

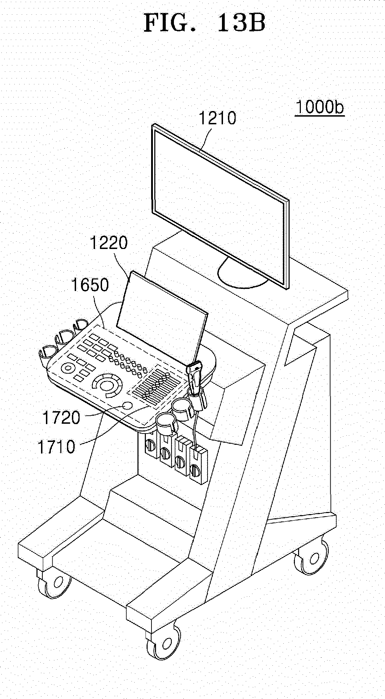

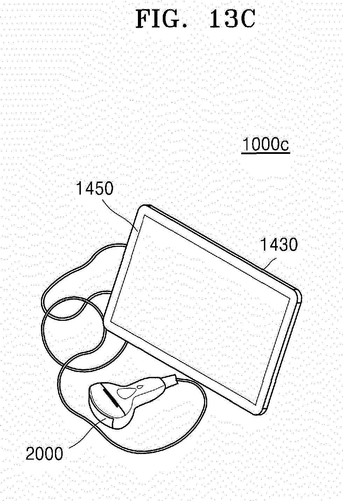

An example of the ultrasound diagnosis apparatus 1000 according to the present exemplary embodiment is described below with reference to FIGS. 13A, 13B, and 13C.

FIGS. 13A, 13B, and 13C are diagrams illustrating ultrasound diagnosis apparatus according to an exemplary embodiment.

Referring to FIGS. 13A and 13B, the ultrasound diagnosis apparatuses 1000a and 1000b may include a main display 1210 and a sub-display 1220. At least one among the main display 1210 and the sub-display 1220 may include a touch screen. The main display 1210 and the sub-display 1220 may display ultrasound images and/or various information processed by the ultrasound diagnosis apparatuses 1000a and 1000b. The main display 1210 and the sub-display 1220 may provide graphical user interfaces (GUI), thereby receiving user's inputs of data to control the ultrasound diagnosis apparatuses 1000a and 1000b. For example, the main display 1210 may display an ultrasound image and the sub-display 1220 may display a control panel to control display of the ultrasound image as a GUI. The sub-display 1220 may receive an input of data to control the display of an image through the control panel displayed as a GUI. The ultrasound diagnosis apparatuses 1000a and 1000b may control the display of the ultrasound image on the main display 1210 by using the input control data.

Referring to FIG. 13B, the ultrasound diagnosis apparatus 1000b may include a control panel 1650. The control panel 1650 may include buttons, trackballs, jog switches, or knobs, and may receive data to control the ultrasound diagnosis apparatus 1000b from the user. For example, the control panel 1650 may include a time gain compensation (TGC) button 1710 and a freeze button 1720. The TGC button 1710 is to set a TGC value for each depth of an ultrasound image. Also, when an input of the freeze button 1720 is detected during scanning an ultrasound image, the ultrasound diagnosis apparatus 1000b may keep displaying a frame image at that time point.

The buttons, trackballs, jog switches, and knobs included in the control panel 1650 may be provided as a GUI to the main display 1210 or the sub-display 1220.

Referring to FIG. 13C, the ultrasound diagnosis apparatus 1000c may include a portable device. An example of the portable ultrasound diagnosis apparatus 1000c may include, for example, smart phones including probes and applications, laptop computers, personal digital assistants (PDAs), or tablet PCs, but an exemplary embodiment is not limited thereto.

The ultrasound diagnosis apparatus 1000c may include the probe 2000 and a main body 1430. The probe 2000 may be connected to one side of the main body 1430 by wire or wirelessly. The main body 1430 may include a touch screen 1450. The touch screen 1450 may display an ultrasound image, various pieces of information processed by the ultrasound diagnosis apparatus 1000c, and a GUI.

The embodiments may be implemented as a software program including instructions stored in a computer-readable storage medium.

A computer may refer to a device configured to retrieve an instruction stored in the computer-readable storage medium and to operate, in response to the retrieved instruction, and may include an ultrasound diagnosis apparatus according to embodiments.

The computer-readable storage medium may be provided in the form of a non-transitory storage medium. In this regard, the term 'non-transitory' means that the storage medium does not include a signal and is tangible, and the term does not distinguish between data that is semi-permanently stored and data that is temporarily stored in the storage medium.

In addition, the ultrasound diagnosis apparatus or the method of controlling the ultrasound diagnosis apparatus according to embodiments may be provided in the form of a computer program product. The computer program product may be traded, as a product, between a seller and a buyer.

The computer program product may include a software program and a computer-readable storage medium having stored thereon the software program. For example, the computer program product may include a product (e.g. a downloadable application) in the form of a software program electronically distributed by a manufacturer of the ultrasound diagnosis apparatus or through an electronic market (e.g., Google ™, Play Store ™, and App Store ™). For such electronic distribution, at least a part of the software program may be stored on the storage medium or may be temporarily generated. In this case, the storage medium may be a storage medium of a server of the manufacturer, a server of the electronic market, or a relay server for temporarily storing the software program.

In a system consisting of a server and a terminal (e.g., the ultrasound diagnosis apparatus), the computer program product may include a storage medium of the server or a storage medium of the terminal. Alternatively, in a case where a third device (e.g., a smartphone) that communicates with the server or the terminal is present, the computer program product may include a storage medium of the third device. Alternatively, the computer program product may include a software program that is transmitted from the server to the terminal or the third device or that is transmitted from the third device to the terminal.

In this case, one of the server, the terminal, and the third device may execute the computer program product, thereby performing the method according to embodiments. Alternatively, at least two of the server, the terminal, and the third device may execute the computer program product, thereby performing the method according to embodiments in a distributed manner.

For example, the server (e.g., a cloud server, an artificial intelligence (AI) server, or the like) may execute the computer program product stored in the server, and may control the terminal to perform the method according to embodiments, the terminal communicating with the server.

As another example, the third device may execute the computer program product, and may control the terminal to perform the method according to embodiments, the terminal communicating with the third device. In more detail, the third device may remotely control the ultrasound diagnosis apparatus to emit X-ray to an object, and to generate an image of an inner part of the object, based on detected radiation which passes the object and is detected in an X-ray detector.

As another example, the third device may execute the computer program product, and may directly perform the method according to embodiments, based on at least one value input from an auxiliary device (e.g., a gantry of CT system). In more detail, the auxiliary device may emit X-ray to an object and may obtain information of radiation which passes the object and is detected in an X-ray detector. The third device may receive an input of signal information about the detected radiation from the auxiliary device, and may generate an image of an inner part of the object, based on the input radiation information.

In a case where the third device executes the computer program product, the third device may download the computer program product from the server, and may execute the downloaded computer program product. Alternatively, the third device may execute the computer program product that is preloaded therein, and may perform the method according to the embodiments.

The above-described embodiments of the present disclosure may be embodied in form of a computer-readable recording medium for storing computer executable command languages and data. The command languages may be stored in form of program codes and, when executed by a processor, may perform a certain operation by generating a certain program module. Also, when executed by a processor, the command languages may perform certain operations of the disclosed embodiments.

While embodiments of the present disclosure have been particularly shown and described with reference to the accompanying drawings, it will be understood by those of ordinary skill in the art that various changes in form and details may be made therein without departing from the scope of the inventive concept as defined by the appended claims. The disclosed embodiments should be considered in descriptive sense only and not for purposes of limitation.

## Claims

1. A method of determining abnormality of a heart of a fetus, the method comprising:
detecting a chest and a spine of the fetus in an ultrasound image of a fetal heart;
receiving a user's input for inputting information regarding left and right directions of the chest of the fetus in the ultrasound image;
detecting a position of the heart in the ultrasound image; and
determining abnormality of the heart of the fetus based on the information about the left and right directions input by the user's input and information about the detected position of the heart.

2. The method of claim 1, wherein the determining of the abnormality of the heart of the fetus comprises determining abnormality in a size of the heart of the fetus based on information about areas of the heart and the chest of the fetus in the ultrasound image.

3. The method of claim 2, wherein the determining of the abnormality in the size of the heart of the fetus comprises:
calculating a ratio of an area of the heart to an area of the chest of the fetus in the ultrasound image; and
determining that the size of the heart is abnormal when the calculated area ratio exceeds 1/3.

4. The method of any of the previous claims, wherein the detecting of the position of the heart comprises detecting a cardiac apex based on the detected heart position, and
the determining of the abnormality of the heart of the fetus comprises determining abnormality of the heart of the fetus based on information about the detected heart position and a position and a direction of the detected cardiac apex.

5. The method of any of the previous claims, wherein the detecting of the position of the heart comprises detecting the position of the heart in the ultrasound image through training of inputting the ultrasound image to a deep neural network (DNN)-based machine learning model.

6. The method of any of the previous claims, further comprising displaying a user interface (UI) indicating information about the abnormality of the heart of the fetus by using at least one of numbers, characters, graphs, images, and color.

7. The method of claim 6, wherein the displaying of the UI comprises displaying a graph of the heart size per week that indicates, on a graph, a ratio of the size of the heart of the fetus to the size of the chest according to pregnancy weeks, and
the graph of the heart size per week comprises a guideline regarding a value corresponding to a heart size in a normal range.

8. An ultrasound diagnosis apparatus for determining abnormality of a heart of a fetus, the ultrasound diagnosis apparatus comprising:
a user input interface configured to receive a user's input for inputting information regarding left and right directions of a chest of the fetus in an ultrasound image related to a fetal heart; and
a controller configured to detect the chest and a spine of the fetus in the ultrasound image, detect a position of the heart in the ultrasound image, and determine abnormality of the heart of the fetus based on the information about the left and right directions input by the user's input and information about the detected position of the heart.

9. The ultrasound diagnosis apparatus of claim 8, wherein the controller is further configured to determine abnormality in a size of the heart of the fetus based on information about areas of the heart and the chest of the fetus in the ultrasound image.

10. The ultrasound diagnosis apparatus of claim 8 or 9, wherein the controller is further configured to calculate a ratio of an area of the heart to an area of the chest of the fetus in the ultrasound image and determine that the size of the heart is abnormal when the calculated area ratio exceeds 1/3.

11. The ultrasound diagnosis apparatus of any of the claims 8 -10, wherein the controller is further configured to detect a cardiac apex based on the detected heart position and determine abnormality of the heart of the fetus based on information about the detected heart position and a position and a direction of the detected cardiac apex.

12. The ultrasound diagnosis apparatus of any of the claims 8-11, wherein the controller comprises a machine learning module for detecting the position of the heart in the ultrasound image through training of inputting the ultrasound image to a deep neural network (DNN)-based machine learning model.

13. The ultrasound diagnosis apparatus of any of the claims 8-12, further comprising a display displaying a user interface (UI) indicating information about the abnormality of the heart of the fetus by using at least one of numbers, characters, graphs, images, and color.

14. The ultrasound diagnosis apparatus of claim 13, wherein the display displays a graph of the heart size per week that indicates, on a graph, a ratio of the size of the heart of the fetus to the size of the chest according to pregnancy weeks, and
the graph of the heart size per week comprises a guideline regarding a value corresponding to a heart size in a normal range.

15. A computer program product comprising a computer-readable storage medium, wherein the storage medium comprising instructions, which, when executed by one or more processors, cause an apparatus to:
detect a chest and a spine of the fetus in an ultrasound image related to a fetal heart;
receive, through a user input interface of the apparatus, a user's input for inputting information about left and right directions of the chest of the fetus in the ultrasound image;
detect a position of the heart in the ultrasound image based on the information about the left and right directions input by the user's input; and
determine abnormality of the heart of the fetus based on information about the detected position of the heart.

## Patentansprüche

1. Verfahren zur Bestimmung einer Anomalie eines fötalen Herzens, welches Folgendes umfasst:
Erfassen des Brustkorbs und der Wirbelsäule des Fötus in einem Ultraschallbild eines fötalen Herzens;
Empfangen einer Benutzereingabe zur Eingabe von Informationen bezüglich der linken und rechten Seite des Brustkorbs des Fötus in dem Ultraschallbild;
Erfassen einer Position des Herzens in dem Ultraschallbild; und
Bestimmen der Anomalie des fötalen Herzens anhand der Informationen bezüglich der linken und rechten Seite, die durch die Benutzereingabe eingegeben wurde, und anhand von Informationen bezüglich der erfassten Position des Herzens.

2. Verfahren nach Anspruch 1, wobei die Bestimmung der Anomalie des fötalen Herzens die Bestimmung einer Größenanomalie des fötalen Herzens anhand von Informationen bezüglich der Herz- und Brustkorbfläche des Fötus in dem Ultraschallbild umfasst.

3. Verfahren nach Anspruch 2, wobei die Bestimmung der Größenanomalie des fötalen Herzens Folgendes umfasst:
Berechnen des Verhältnisses der Herzfläche zur Brustkorbfläche des Fötus in dem Ultraschallbild; und
Feststellen, dass die Herzgröße abnormal ist, wenn das berechnete Flächenverhältnis 1/3 überschreitet.

4. Verfahren nach einem der vorhergehenden Ansprüche, wobei das Erfassen der Herzposition das Erfassen der Herzspitze anhand der erfassten Herzposition umfasst, und
das Bestimmen der Anomalie des fötalen Herzens das Bestimmen der Anomalie des fötalen Herzens anhand von Informationen bezüglich der erfassten Herzposition und bezüglich der Position und Richtung der erfassten Herzspitze umfasst.

5. Verfahren nach einem der vorhergehenden Ansprüche, wobei das Erfassen der Herzposition das Erfassen der Herzposition in dem Ultraschallbild durch Training der Eingabe des Ultraschallbildes in ein auf einem tiefen neuronalen Netzwerk (DNN, Deep Neural Network) basierendes maschinelles Lernmodell umfasst.

6. Verfahren nach einem der vorhergehenden Ansprüche, welches ferner das Anzeigen einer Benutzeroberfläche (UI, User Interface) umfasst, die Informationen bezüglich der Anomalie des fötalen Herzens anzeigt, indem mindestens eine der folgenden Optionen verwendet wird: Zahlen, Zeichen, Graphen, Bilder und Farbe.

7. Verfahren nach Anspruch 6, wobei das Anzeigen der UI das Anzeigen eines Graphen der wöchentlichen Herzgröße umfasst, der das Verhältnis der Herzgröße zur Brustkorbgröße des Fötus entsprechend der Schwangerschaftswochen auf einem Graphen anzeigt, und
der Graph der wöchentlichen Herzgröße eine Referenzlinie eines Wertes einer Herzgröße im Normbereich umfasst.

8. Ultraschall-Diagnosevorrichtung zur Bestimmung einer Anomalie eines fötalen Herzens, wobei die Ultraschall-Diagnosevorrichtung Folgendes umfasst:
eine Benutzereingabeschnittstelle, die so konfiguriert ist, dass sie eine Benutzereingabe zur Eingabe von Informationen bezüglich der linken und rechten Seite des Brustkorbs des Fötus in einem das fötale Herz betreffenden Ultraschallbild empfängt; und
eine Steuerung, die so konfiguriert ist, dass sie den Brustkorb und die Wirbelsäule des Fötus in dem Ultraschallbild erfasst, eine Position des Herzens in dem Ultraschallbild erfasst und eine Anomalie des fötalen Herzens anhand der durch die Benutzereingabe eingegebenen Informationen bezüglich der linken und rechten Seite und anhand von Informationen bezüglich der erfassten Position des Herzens bestimmt.

9. Ultraschall-Diagnosevorrichtung nach Anspruch 8, wobei die Steuerung ferner so konfiguriert ist, dass sie eine Anomalie der Herzgröße des Fötus anhand von Informationen bezüglich der Herz- und Brustkorbfläche des Fötus in dem Ultraschallbild bestimmt.

10. Ultraschall-Diagnosevorrichtung nach Anspruch 8 oder 9, wobei die Steuerung ferner konfiguriert ist, um das Verhältnis der Herzfläche zur Brustkorbfläche des Fötus in dem Ultraschallbild zu berechnen und zu bestimmen, dass die Herzgröße abnormal ist, wenn das berechnete Flächenverhältnis 1/3 überschreitet.

11. Ultraschall-Diagnosevorrichtung nach einem der Ansprüche 8 bis 10, wobei die Steuerung ferner so konfiguriert ist, dass sie die Herzspitze anhand der erfassten Herzposition erfasst und eine Anomalie des fötalen Herzens anhand von Informationen bezüglich der erfassten Herzposition und der Position und Richtung der erfassten Herzspitze bestimmt.

12. Ultraschall-Diagnosevorrichtung nach einem der Ansprüche 8 bis 11, wobei die Steuerung ein maschinelles Lernmodul zur Erfassung der Herzposition in dem Ultraschallbild durch Training der Eingabe des Ultraschallbildes in ein auf einem tiefen neuronalen Netzwerk (DNN, Deep Neural Network) basierendes maschinelles Lernmodell umfasst.

13. Ultraschall-Diagnosevorrichtung nach einem der Ansprüche 8 bis 12, die ferner ein Display zum Anzeigen einer Benutzeroberfläche (UI, User Interface) umfasst, auf dem Informationen bezüglich der Anomalie des fötalen Herzens angezeigt werden, indem mindestens eine der folgenden Optionen verwendet wird: Zahlen, Zeichen, Graphen, Bilder und Farbe.

14. Ultraschall-Diagnosevorrichtung nach Anspruch 13, wobei auf dem Display ein Graph der wöchentlichen Herzgröße angezeigt wird, der das Verhältnis der Herzgröße zur Brustkorbgröße des Fötus entsprechend den Schwangerschaftswochen auf einem Graphen anzeigt, und
der Graph der wöchentlichen Herzgröße eine Referenzlinie eines Wertes einer Herzgröße im Normbereich umfasst.

15. Computerprogrammprodukt, welches ein computerlesbares Speichermedium umfasst, wobei das Speichermedium Anweisungen umfasst, die, wenn sie von einem oder mehreren Prozessoren ausgeführt werden, eine Vorrichtung veranlassen:
den Brustkorb und die Wirbelsäule des Fötus in einem das fötale Herz betreffenden Ultraschallbild zu erfassen;
über eine Benutzereingabeschnittstelle der Vorrichtung eine Benutzereingabe zur Eingabe von Informationen bezüglich der linken und rechten Seite des Brustkorbs des Fötus in dem Ultraschallbild zu empfangen;
eine Position des Herzens in dem Ultraschallbild anhand der Informationen bezüglich der linken und rechten Seite zu erfassen, die über die Benutzereingabe eingegeben wurden; und
eine Anomalie des fötalen Herzens anhand von Informationen bezüglich der erfassten Position des Herzens festzustellen.

## Revendications

1. Procédé de détermination d'une anomalie du cœur d'un fœtus, le procédé comprenant les opérations consistant à :
détecter le thorax et la colonne vertébrale du fœtus dans une image échographique d'un cœur fœtal, recevoir une entrée utilisateur permettant de saisir des informations concernant les directions gauche et droite du thorax du fœtus dans l'image échographique,
détecter la position du cœur dans l'image échographique, et
déterminer une anomalie du cœur du fœtus compte tenu des informations concernant les directions gauche et droite saisies par l'entrée utilisateur et d'informations concernant la position détectée du cœur.

2. Procédé selon la revendication 1, dans lequel la détermination de l'anomalie du cœur du fœtus comprend la détermination d'une anomalie concernant la taille du cœur du fœtus compte tenu d'informations concernant des régions cardiaque et thoracique du fœtus dans l'image échographique.

3. Procédé selon la revendication 2, dans lequel la détermination de l'anomalie concernant la taille du cœur du fœtus comprend les opérations consistant à :
calculer un rapport entre une région cardiaque et une région thoracique du fœtus dans l'image échographique, et
déterminer que la taille du cœur est anormale lorsque le rapport calculé est supérieur à 1/3.

4. Procédé selon l'une quelconque des revendications précédentes, dans lequel la détection de la position du cœur comprend la détection de l'apex du cœur compte tenu de la position détectée du cœur, et
la détermination de l'anomalie du cœur du fœtus comprend la détermination de l'anomalie du cœur du fœtus compte tenu d'informations concernant la position détectée du cœur ainsi que la position et la direction de l'apex du cœur ayant été détecté.

5. Procédé selon l'une quelconque des revendications précédentes, dans lequel la détection de la position du cœur comprend la détection de la position du cœur dans l'image échographique par apprentissage effectué par l'introduction de l'image échographique dans un modèle d'apprentissage automatique reposant sur un réseau de neurones profonds.

6. Procédé selon l'une quelconque des revendications précédentes, comprenant en outre l'affichage d'une interface utilisateur (IU) communiquant des informations concernant l'anomalie du cœur du fœtus au moyen de nombres, de caractères, de graphiques, d'images et/ou de couleurs.

7. Procédé selon la revendication 6, dans lequel l'affichage de l'IU comprend l'affichage d'un graphique de la taille du cœur par semaine qui indique, sur un graphique, le rapport entre la taille du cœur du fœtus et la taille du thorax en fonction du nombre de semaines de grossesse, et
le graphique de la taille du cœur par semaine comprend une ligne indicatrice concernant une valeur correspondant à la taille d'un cœur situé dans une plage normale.

8. Appareil d'échographie diagnostique permettant de déterminer une anomalie du cœur d'un fœtus, l'appareil d'échographie diagnostique comprenant :
une interface d'entrée utilisateur conçue pour recevoir une entrée utilisateur permettant de saisir des informations concernant les directions gauche et droite du thorax du fœtus dans une image échographique se rapportant à un cœur fœtal, et
un contrôleur conçu pour détecter le thorax et la colonne vertébrale du fœtus dans l'image échographique, pour détecter la position du cœur dans l'image échographique et pour déterminer une anomalie du cœur du fœtus compte tenu des informations concernant les directions gauche et droite saisies par l'entrée utilisateur et d'informations concernant la position détectée du cœur.

9. Appareil d'échographie diagnostique selon la revendication 8, dans lequel le contrôleur est en outre conçu pour déterminer une anomalie concernant la taille du cœur du fœtus compte tenu d'informations concernant des régions cardiaque et thoracique du fœtus dans l'image échographique.

10. Appareil d'échographie diagnostique selon la revendication 8 ou 9, dans lequel le contrôleur est en outre conçu pour calculer un rapport entre une région cardiaque et une région thoracique du fœtus dans l'image échographique et pour déterminer que la taille du cœur est anormale lorsque le rapport calculé est supérieur à 1/3.

11. Appareil d'échographie diagnostique selon l'une quelconque des revendications 8 à 10, dans lequel le contrôleur est en outre conçu pour détecter l'apex du cœur compte tenu de la position du cœur détectée et pour déterminer une anomalie du cœur du fœtus compte tenu d'informations concernant la position détectée du cœur ainsi que la position et la direction de l'apex du cœur ayant été détecté.

12. Appareil d'échographie diagnostique selon l'une quelconque des revendications 8 à 11, dans lequel le contrôleur comprend un module d'apprentissage automatique permettant de détecter la position du cœur dans l'image échographique grâce à un apprentissage effectué par l'introduction de l'image échographique dans un modèle d'apprentissage automatique reposant sur un réseau de neurones profonds.

13. Appareil d'échographie diagnostique selon l'une quelconque des revendications 8 à 12, comprenant en outre un écran affichant une interface utilisateur (IU) communiquant des informations concernant l'anomalie du cœur du fœtus au moyen de nombres, de caractères, de graphiques, d'images et/ou de couleurs.

14. Appareil d'échographie diagnostique selon la revendication 13, dans lequel l'écran affiche un graphique de la taille du cœur par semaine qui indique, sur un graphique, le rapport entre la taille du cœur du fœtus et la taille du thorax en fonction du nombre de semaines de grossesse, et
le graphique de la taille du cœur par semaine comprend une ligne indicatrice concernant une valeur correspondant à la taille d'un cœur situé dans une plage normale.

15. Produit-programme informatique comprenant un support de stockage lisible par ordinateur, ledit support de stockage comprenant des instructions qui, lorsqu'elles sont exécutées par un ou plusieurs processeurs, amènent un appareil à :
détecter le thorax et la colonne vertébrale d'un fœtus dans une image échographique se rapportant à un cœur fœtal,
recevoir, par le biais d'une interface d'entrée utilisateur de l'appareil, une entrée utilisateur permettant de saisir des informations concernant les directions gauche et droite du thorax du fœtus dans l'image échographique,
détecter la position du cœur dans l'image échographique compte tenu des informations concernant les directions gauche et droite saisies par l'entrée utilisateur, et
déterminer une anomalie du cœur du fœtus compte tenu d'informations concernant la position détectée du cœur.
